# EUROPÄISCHE PATENTANMELDUNG

(11) **EP 4 455 996 A1**
(43) Veröffentlichungstag der Anmeldung: **30.10.2024**
(21) Anmeldenummer: 23170624.3
(22) Anmeldetag: 28.04.2023
(51) Int. Cl.: G06T 11/00

(54) **BEREITSTELLEN EINES 3D-ERGEBNISDATENSATZES**

(71) Anmelder: Siemens Healthineers AG, 91301 Forchheim (DE)
(72) Erfinder: Dr. Hornung, Oliver, 91364 Unterleinleiter (DE); Dr. Kowarschik, Markus, 90408 Nürnberg (DE); Dr. Manhart, Michael, 90765 Fürth (DE); Meier, Manuela, 90425 Nürnberg (DE)
(74) Vertreter: Siemens Healthineers Patent Attorneys

(57) **Zusammenfassung**

Die Erfindung betrifft ein computerimplementiertes Verfahren zum Bereitstellen eines 3D-Ergebnisdatensatzes, umfassend:
- Erfassen von Projektionsabbildungen eines Untersuchungsobjekts, welche mittels eines medizinischen Röntgengeräts aus verschiedenen Projektionsrichtungen aufgenommen sind,
- Bereitstellen einer initialen Projektionsmatrix basierend auf einem statischen Modell des Röntgengeräts,
- Bereitstellen einer weiteren Projektionsmatrix durch Anwenden einer trainierten Funktion auf Eingabedaten,
wobei die Eingabedaten auf der initialen Projektionsmatrix und den Projektionsabbildungen basieren,
wobei zumindest ein Parameter der trainierten Funktion basierend auf einer Bildqualitätsmetrik und/oder einer Konsistenzmetrik angepasst ist,
wobei die weitere Projektionsmatrix als Ausgabedaten der trainierten Funktion bereitgestellt wird,

- Bereitstellen des 3D-Ergebnisdatensatzes durch Rekonstruktion aus den Projektionsabbildungen mittels der weiteren Projektionsmatrix.

Die Erfindung betrifft weiterhin ein computerimplementiertes Verfahren zum Bereitstellen einer trainierten Funktion, eine Bereitstellungseinheit, ein medizinisches Röntgengerät, eine Trainingseinheit und ein Computerprogrammprodukt.

## Beschreibung

Die vorliegende Erfindung betrifft ein computerimplementiertes Verfahren zum Bereitstellen eines 3D-Ergebnisdatensatzes, ein computerimplementiertes Verfahren zum Bereitstellen einer trainierten Funktion, eine Bereitstellungseinheit, ein medizinisches Röntgengerät, eine Trainingseinheit und ein Computerprogrammprodukt.

Zur dreidimensionalen (3D) Rekonstruktion in einer Kegelstrahlcomputertomographie (engl. cone-beam computed tomography, CBCT) eines Untersuchungsobjekts aus mehreren Projektionsrichtungen, wird häufig zunächst eine "offline" Kalibrierung mittels eines Phantoms bekannter Geometrie durchgeführt. Anhand von zweidimensionalen (2D) Projektionsabbildungen des Phantoms können 2D/3D-Korrespondenzen bestimmt und zur Abschätzung von Projektionsmatrizen für jede einzelne Projektionsrichtung verwendet werden. Dabei können die Projektionsmatrizen beispielsweise in einer Datenbank abgespeichert werden. Bei der späteren Aufnahme von Projektionsabbildungen eines unbekannten rigiden Untersuchungsobjekts aus den gleichen Projektionsrichtungen können die Projektionsmatrizen aus der Datenbank für die 3D-Rekonstruktion ausgelesen werden.

Die Verwendung der zuvor ermittelten Projektionsmatrizen für unbekannte Untersuchungsobjekte erfordert nachteilig eine hohe Reproduzierbarkeit einer Bildkette und von Bewegungen des Bildgebungsgeräts. Hiermit sind hohe Anforderungen an die Entwicklung und das mechanische Design von technischen Systemkomponenten des Bildgebungsgeräts verbunden. Eine Änderung von Systemkomponenten des Bildgebungsgeräts, beispielsweise einer Röntgenröhre, einem Antrieb, einem Getriebe und/oder einem Röntgendetektor, erfordert eine vollständige Wiederholung der Kalibrierung. Die Kalibrierung ist zeit- und kostenintensiv und ist zudem für alle Projektionsrichtungen von 3D-Aufnahmeprotokollen erforderlich. Die 3D-Aufnahme von Projektionsabbildungen ist zudem nachteilig an eine kalibrierte Isozentrumsposition und die kalibrierte Aufnahmetrajektorie, insbesondere die Projektionsrichtungen, gebunden.

Es ist daher die Aufgabe der vorliegenden Erfindung, eine flexiblere Aufnahme von Projektionsabbildungen eines Untersuchungsobjekts und verbesserte 3D-Rekonstruktion aus den Projektionsabbildungen zu ermöglichen.

Die Aufgabe wird erfindungsgemäß gelöst durch den Gegenstand der unabhängigen Ansprüche. Vorteilhafte Ausführungsformen mit zweckmäßigen Weiterbildungen sind Gegenstand der Unteransprüche. Unabhängig vom grammatikalischen Geschlecht eines bestimmten Begriffes sind Personen mit männlicher, weiblicher oder anderer Geschlechteridentität mit umfasst.

Nachstehend wird die erfindungsgemäße Lösung der Aufgabe sowohl in Bezug auf Verfahren und Vorrichtungen zum Bereitstellen eines 3D-Ergebnisdatensatzes als auch in Bezug auf Verfahren und Vorrichtungen zum Bereitstellen einer trainierten Funktion beschrieben. Hierbei können Merkmale, Vorteile und alternative Ausführungsformen von Datenstrukturen und/oder Funktionen bei Verfahren und Vorrichtungen zum Bereitstellen eines 3D-Ergebnisdatensatzes auf analoge Datenstrukturen und/oder Funktionen bei Verfahren und Vorrichtungen zum Bereitstellen einer trainierten Funktion übertragen werden. Analoge Datenstrukturen können hierbei insbesondere durch die Verwendung der Vorsilbe "Trainings" gekennzeichnet sein. Weiterhin können die in Verfahren und Vorrichtungen zur Prozedurunterstützung verwendeten trainierten Funktionen insbesondere durch Verfahren und Vorrichtungen zum Bereitstellen einer trainierten Funktion angepasst und/oder bereitgestellt worden sein.

Die Erfindung betrifft in einem ersten Aspekt ein computerimplementiertes Verfahren zum Bereitstellen eines 3D-Ergebnisdatensatzes. In einem ersten Schritt werden Projektionsabbildungen eines Untersuchungsobjekts erfasst, welche mittels eines medizinischen Röntgengeräts, insbesondere entlang einer 3D-Trajektorie, aus verschiedenen Projektionsrichtungen aufgenommen sind. Ferner wird eine initiale Projektionsmatrix basierend auf einem statischen Modell des Röntgengeräts bereitgestellt. Zudem wird eine weitere Projektionsmatrix durch Anwenden einer trainierten Funktion auf Eingabedaten bereitgestellt. Dabei basieren die Eingabedaten der trainierten Funktion auf der initialen Projektionsmatrix und den Projektionsabbildungen. Ferner ist zumindest ein Parameter der trainierten Funktion basierend auf einer Bildqualitätsmetrik und/oder einer Konsistenzmetrik angepasst. Die weitere Projektionsmatrix wird als Ausgabedaten der trainierten Funktion bereitgestellt. Hiernach wird der 3D-Ergebnisdatensatz durch Rekonstruktion aus den Projektionsabbildungen mittels der weiteren Projektionsmatrix bereitgestellt.

Die vorstehend beschriebenen Schritte des vorgeschlagenen Verfahrens können teilweise oder vollständig computerimplementiert sein. Zudem können die vorstehend beschriebenen Schritte des vorgeschlagenen Verfahrens zumindest teilweise, insbesondere vollständig, nacheinander oder zumindest teilweise gleichzeitig ausgeführt werden.

Das Erfassen der Projektionsabbildungen kann ein Empfangen und/oder Aufnehmen der Projektionsabbildungen umfassen. Das Empfangen der Projektionsabbildungen kann insbesondere ein Erfassen und/oder Auslesen eines computerlesbaren Datenspeichers und/oder ein Empfangen aus einer Datenspeichereinheit, beispielsweise einer Datenbank, umfassen, beispielsweise mittels einer Schnittstelle.

Das Untersuchungsobjekt kann beispielsweise eine menschliche und/oder tierische Patientin und/oder ein menschlicher und/oder tierischer Patient und/oder ein Untersuchungsphantom, insbesondere ein Gefäßphantom, sein.

Die Projektionsabbildungen sind mittels des medizinischen Röntgengeräts, insbesondere einem medizinischen C-Bogen-Röntgengerät, entlang einer 3D-Trajektorie aus verschiedenen Projektionsrichtungen aufgenommen. Die verschiedenen Projektionsrichtungen können vorteilhafterweise zumindest teilweise nicht kollinear sein. Ferner können die Projektionsrichtungen jeweils einen Verlauf eines Strahls, insbesondere eines Zentral- und/oder Mittenstrahls, zwischen einer Röntgenquelle und einem Röntgendetektor, insbesondere einem Detektormittelpunkt, des Röntgengeräts zum Aufnahmezeitpunkt der jeweiligen Projektionsabbildungen beschreiben. Insbesondere können die Projektionsrichtungen jeweils eine Angulation des Röntgengeräts bezüglich des Untersuchungsobjekts und/oder eines Isozentrums, insbesondere Drehzentrums, einer definierten Anordnung von Röntgenquelle und Röntgendetektor beschreiben. Dabei kann das Isozentrum einen räumlichen Punkt beschreiben, um welchen die definierte Anordnung von Röntgenquelle und Röntgendetektor bewegbar, insbesondere rotierbar, ist, insbesondere während der Aufnahme der Projektionsabbildungen. Vorteilhafterweise können die verschiedenen Projektionsrichtungen jeweils durch das, insbesondere gemeinsame, Isozentrum verlaufen. Die 3D-Trajektorie kann einen räumlichen Pfad zur Anordnung eines Referenzpunkts, beispielsweise eines Fokuspunkts, der definierten Anordnung von Röntgenquelle und Röntgendetektor beschreiben. Vorteilhafterweise können die mehreren Projektionsabbildungen das Untersuchungsobjekt jeweils 2D räumlich aufgelöst abbilden.

Die zumindest eine initiale Projektionsmatrix, insbesondere mehrere initiale Projektionsmatrizen, werden basierend auf einem statischen Modell des Röntgengeräts bereitgestellt. Das statische Modell kann eine physikalische und/oder mathematische Repräsentation statischer Effekte des Röntgengeräts umfassen, beispielsweise ein digitaler Zwilling und/oder ein computergestütztes Modell (engl. computer-aided design, CAD). Das statische Modell kann anhand geometrischer Merkmale des Röntgengeräts und seiner Komponenten bestimmt sein. Vorteilhafterweise kann das statische Modell empfangen werden. Das Empfangen des statischen Modells kann insbesondere ein Erfassen und/oder Auslesen eines computerlesbaren Datenspeichers und/oder ein Empfangen aus einer Datenspeichereinheit, beispielsweise einer Datenbank, umfassen, beispielsweise mittels einer Schnittstelle. Anhand des statischen Modells des Röntgengeräts kann eine Abbildungsgeometrie zwischen der Röntgenquelle und dem Röntgendetektor bestimmt werden. Die initiale Projektionsmatrix kann vorteilhafterweise anhand der Abbildungsgeometrie, insbesondere in Abhängigkeit der jeweiligen Projektionsrichtung, bestimmt werden. Dabei kann die initiale Projektionsmatrix eine Abbildungsvorschrift zur Abbildung von 3D-Objektpunkten des Untersuchungsobjekts auf 2D-Bildpunkte der Projektionsabbildungen umfassen.

Durch das Anwenden der trainierten Funktion auf die Eingabedaten wird die weitere Projektionsmatrix bereitgestellt. Die trainierte Funktion kann vorteilhafterweise durch ein Verfahren des Maschinenlernens trainiert sein. Die trainierte Funktion kann ein, insbesondere künstliches, neuronales Netzwerk, insbesondere ein faltendes neuronales Netzwerk (engl. convolutional neuronal network, CNN) bzw. ein Netzwerk umfassend eine Faltungsschicht (engl. convolutional layer) sein.

Die trainierte Funktion bildet Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder mehrerer Parameter der trainierten Funktion kann insbesondere auf einem Paar aus Trainingseingabedaten und zugehörigen Trainingsausgabedaten, insbesondere Vergleichsausgabedaten, basieren, wobei die trainierte Funktion zur Erzeugung von Trainingsabbildungsdaten auf die Trainingseingabedaten angewendet wird. Insbesondere können das Bestimmen und/oder das Anpassen auf einem Vergleich der Trainingsabbildungsdaten und der Trainingsausgabedaten, insbesondere Vergleichsausgabedaten, basieren. Im Allgemeinen wird auch eine trainierbare Funktion, das heißt, eine Funktion mit noch nicht angepassten einen oder mehreren Parametern, als trainierte Funktion bezeichnet. Durch das Anpassen des einen oder der mehreren Parameter der trainierten Funktion, insbesondere ein Training der trainierten Funktion, kann die trainierte Funktion zur Anpassung an neue Umstände und zur Erkennung und Extrapolation von Mustern ausgebildet werden.

Die trainierte Funktion kann mittels überwachten Lernens, teilüberwachten Lernens, unüberwachten Lernens, verstärkenden Lernens, Repräsentationslernens (engl. representation learning) und/oder aktiven Lernens angepasst werden. Der zumindest eine Parameter der trainierten Funktion kann iterativ durch mehrere Trainingsschritte angepasst werden.

Andere Begriffe für trainierte Funktionen sind trainierte Abbildungsvorschrift, Abbildungsvorschrift mit trainierten Parametern, Funktion mit trainierten Parametern, Algorithmus des maschinellen Lernens. Ein Beispiel für eine trainierte Funktion ist ein künstliches neuronales Netzwerk, wobei Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion ein neuronales Netzwerk, beispielsweise ein tiefes künstliches neuronales Netzwerk (engl. deep neuronal network, deep artificial neural network) und/oder ein faltendes neuronales Netzwerk und/oder ein tiefes faltendes Netzwerk und/oder ein adversariales Netzwerk und/oder ein tiefes adversariales Netzwerk und/oder ein generatives adversariales Netzwerk, eine "Support Vector Machine", ein Entscheidungsbaum und/oder ein Bayessches Netzwerk umfassen. Alternativ oder zusätzlich kann die trainierte Funktion auf einem k-Means-Algorithmus (engl. k-means clustering), Q-Lernen, genetischen Algorithmen und/oder einer Assoziationsanalyse (engl. association rules) basieren.

Die trainierte Funktion kann insbesondere mittels einer Rückpropagation trainiert sein. Zunächst können Trainingsabbildungsdaten durch Anwendung der trainierten Funktion auf die Trainingseingabedaten bestimmt werden. Hiernach kann eine Abweichung zwischen den Trainingsabbildungsdaten und den Trainingsausgabedaten, insbesondere den Vergleichsausgabedaten, durch Anwenden einer Fehlerfunktion auf die Trainingsabbildungsdaten und die Trainingsausgabedaten, insbesondere die Vergleichsausgabedaten, ermittelt werden. Ferner kann zumindest ein Parameter, insbesondere eine Gewichtung der trainierten Funktion iterativ angepasst werden. Hierdurch kann die Abweichung zwischen den Trainingsabbildungsdaten und den Trainingsausgabedaten, insbesondere den Vergleichsausgabedaten, während des Trainings der trainierten Funktion minimiert werden.

Vorteilhafterweise weist die trainierte Funktion, insbesondere das neuronale Netzwerk, eine Eingabeschicht und eine Ausgabeschicht auf. Dabei kann die Eingabeschicht zum Empfangen von Eingabedaten ausgebildet sein. Ferner kann die Ausgabeschicht zum Bereitstellen von Abbildungsdaten, insbesondere den Ausgabedaten, ausgebildet sein. Dabei können die Eingabeschicht und/oder die Ausgabeschicht jeweils mehrere Kanäle, insbesondere Neuronen, umfassen.

Die Eingabedaten der trainierten Funktion basieren auf den Prozedurdaten. Insbesondere umfassen die Eingabedaten der trainierten Funktion die Prozedurdaten. Ferner stellt die trainierte Funktion die Zeitinformation und die Zielprozedurkonfiguration als Ausgabedaten bereit. Zumindest ein Parameter der trainierten Funktion ist basierend auf einem Vergleich einer Trainingsprozedurkonfiguration mit einer Vergleichsprozedurkonfiguration und einem Vergleich einer Trainingszeitinformation mit einer Vergleichszeitinformation angepasst. Insbesondere kann die trainierte Funktion durch eine Ausführungsform des vorgeschlagenen Verfahrens zum Bereitstellen einer trainierten Funktion bereitgestellt werden, welches im weiteren Verlauf beschrieben ist.

Die Eingabedaten der trainierten Funktion basieren auf der initialen Projektionsmatrix und den Projektionsabbildungen. Insbesondere umfassen die Eingabedaten der trainierten Funktion die initiale Projektionsmatrix und die Projektionsabbildungen. Ferner stellt die trainierte Funktion die weitere Projektionsmatrix als Ausgabedaten bereit. Zumindest ein Parameter der trainierten Funktion ist basierend auf einer Bildqualitätsmetrik und/oder einer Konsistenzmetrik angepasst. Insbesondere kann die trainierte Funktion durch eine Ausführungsform des vorgeschlagenen Verfahrens zum Bereitstellen einer trainierten Funktion bereitgestellt werden, welches im weiteren Verlauf beschrieben ist. Dabei kann die weitere Projektionsmatrix eine Abbildungsvorschrift zur Abbildung von 3D-Objektpunkten des Untersuchungsobjekts auf 2D-Bildpunkte der Projektionsabbildungen umfassen. Vorteilhafterweise wird der 3D-Ergebnisdatensatz aus den mehreren Projektionsabbildungen mittels der weiteren Projektionsmatrix rekonstruiert, beispielsweise mittels einer gefilterten Rückprojektion.

Dabei kann der 3D-Ergebnisdatensatz mehrere Bildpunkte, insbesondere Voxel, mit Bildwerten, beispielsweise Intensitätswerten und/oder Schwächungswerten, welche das Untersuchungsobjekt 3D räumlich aufgelöst abbilden.

Das Bereitstellen des 3D-Ergebnisdatensatzes kann ein Speichern auf einem computerlesbaren Speichermedium und/oder ein Anzeigen auf einer Darstellungseinheit und/oder ein Übertragen an eine Bereitstellungseinheit umfassen. Insbesondere kann eine graphische Darstellung des 3D-Ergebnisdatensatzes mittels der Darstellungseinheit angezeigt werden.

Das vorgeschlagene Verfahren kann vorteilhaft eine flexiblere Aufnahme der Projektionsabbildungen des Untersuchungsobjekts und eine verbesserte 3D-Rekonstruktion des 3D-Ergebnisdatensatzes aus den Projektionsabbildungen ermöglichen.

In einer weiteren vorteilhaften Ausführungsform des vorgeschlagenen Verfahrens zum Bereitstellen eines 3D-Ergebnisdatensatzes können jeweils eine initiale und eine weitere Projektionsmatrix zu den verschiedenen Projektionsrichtungen bereitgestellt werden. Dabei können die Eingabedaten der trainierten Funktion auf den initialen Projektionsmatrizen basieren. Ferner kann der 3D-Ergebnisdatensatz durch Rekonstruktion aus den Projektionsabbildungen mittels der weiteren Projektionsmatrizen bereitgestellt werden.

Vorteilhafterweise kann zu jeder verschiedenen Projektionsrichtung der mehreren Projektionsrichtungen jeweils eine initiale Projektionsmatrix basierend auf dem statischen Modell des Röntgengeräts bereitgestellt werden. Die Eingabedaten der trainierten Funktion können vorteilhafterweise auf den mehreren initialen Projektionsmatrizen basieren, insbesondere die mehreren initialen Projektionsmatrizen umfassen. Ferner kann die trainierte Funktion zu jeder verschiedenen Projektionsrichtung der mehreren Projektionsrichtungen jeweils eine weitere Projektionsmatrix als Ausgabedaten bereitstellen. Vorteilhafterweise kann der 3D-Ergebnisdatensatz aus den mehreren Projektionsabbildungen rekonstruiert werden, wobei die mit der Projektionsrichtung der jeweiligen Projektionsabbildung korrespondierende weitere Projektionsmatrix verwendet wird.

Die vorgeschlagene Ausführungsform kann eine verbesserte, insbesondere an die verschiedenen Projektionsrichtungen angepasste, Rekonstruktion des 3D-Ergebnisdatensatzes ermöglichen.

In einer weiteren vorteilhaften Ausführungsform des vorgeschlagenen Verfahrens zum Bereitstellen eines 3D-Ergebnisdatensatzes können die Eingabedaten der trainierten Funktion zusätzlich auf dem statischen Modell des Röntgengeräts basieren.

Vorteilhafterweise können die Eingabedaten der trainierten Funktion zusätzlich das statische Modell des Röntgengeräts umfassen. Hierdurch kann vorteilhaft die wenigstens eine weitere Projektionsmatrix zusätzlich unter Berücksichtigung des statischen Modells des Röntgengeräts bereitgestellt werden.

In einer weiteren vorteilhaften Ausführungsform des vorgeschlagenen Verfahrens zum Bereitstellen eines 3D-Ergebnisdatensatzes kann eine Information zu dynamischen Bewegungsfreiheitsgraden des Röntgengeräts erfasst werden, welche einen latenten Raum definiert. Dabei können die Eingabedaten der trainierten Funktion zusätzlich auf dem latenten Raum basieren.

Das medizinische Röntgengerät kann mehrere dynamische, insbesondere nicht-lineare, Bewegungsfreiheitsgrade aufweisen. Die dynamischen Bewegungsfreiheitsgrade können beispielsweise eine Wirkung von Reibung und/oder Dämpfung und/oder Schwingungen und/oder Trägheitsmomenten und/oder Zentrifugaleffekten und/oder Corioliseffekten auf eine Bewegung des Röntgengeräts kennzeichnen. Insbesondere können die dynamischen Bewegungsfreiheitsgrade einen maximalen Einfluss von Reibung und/oder Dämpfung und/oder Schwingungen und/oder Trägheitsmomenten und/oder Zentrifugaleffekten und/oder Corioliseffekten auf die Bewegung des Röntgengeräts beschreiben. Der latente Raum kann durch die dynamischen Bewegungsfreiheitsgrade, insbesondere auch durch statische Bewegungsfreiheitsgrade, des Röntgengeräts definiert, insbesondere vorgegeben und/oder begrenzt, werden. Die dynamischen Bewegungsfreiheitsgrade, insbesondere der latente Raum, können physikalisch mögliche dynamische Abweichungen von statischen Bewegungen des Röntgengeräts beschreiben.

Die dynamischen Bewegungsfreiheitsgrade, insbesondere der latente Raum, des Röntgengeräts können gelernt sein, beispielsweise mittels eines Variationsautoencoders (engl. variational autoencoder). Die dynamischen Bewegungsfreiheitsgrade, insbesondere der latente Raum, können vorteilhafterweise anhand von Abweichungen zwischen initialen Projektionsmatrizen, welche anhand des statischen Modells des Röntgengeräts bestimmt sind, und Kalibrierprojektionsmatrizen, welche anhand von Kalibrierprojektionsabbildungen eines Phantoms bekannter Geometrie bestimmt sind, ermittelt werden.

Vorteilhafterweise können die Eingabedaten der trainierten Funktion zusätzlich den latenten Raum, insbesondere eine den latenten Raum charakterisierende Information, umfassen. Hierdurch kann vorteilhaft die wenigstens eine weitere Projektionsmatrix unter zusätzlicher Berücksichtigung der dynamischen Bewegungsfreiheitsgrade des Röntgengeräts bereitgestellt werden.

Die Erfindung betrifft in einem zweiten Aspekt ein computerimplementiertes Verfahren zum Bereitstellen einer trainierten Funktion. Dabei werden Trainingsprojektionsabbildungen eines Trainingsuntersuchungsobjekts erfasst, welche das Trainingsuntersuchungsobjekt, insbesondere entlang einer 3D-Trajektorie, aus verschiedenen Projektionsrichtungen abbilden. Dabei sind die Trainingsprojektionsabbildungen simuliert oder mittels eines medizinischen Trainingsröntgengeräts aufgenommen. In einem weiteren Schritt wird eine initiale Trainingsprojektionsmatrix basierend auf einem statischen Trainingsmodell des medizinischen Trainingsröntgengeräts bereitgestellt. Ferner wird eine weitere Trainingsprojektionsmatrix durch Anwenden der trainierten Funktion auf Eingabedaten bereitgestellt. Dabei basieren die Eingabedaten der trainierten Funktion auf den Trainingsprojektionsabbildungen und der initialen Trainingsprojektionsmatrix. Die weitere Trainingsprojektionsmatrix wird als Ausgabedaten der trainierten Funktion bereitgestellt. In einem weiteren Schritt wird ein 3D-Trainingsdatensatz aus den Trainingsprojektionsabbildungen mittels der weiteren Trainingsprojektionsmatrix rekonstruiert. Ferner wird jeweils ein Bewertungsparameter durch Anwenden einer Bildqualitätsmetrik und/oder einer Konsistenzmetrik auf den 3D-Trainingsdatensatz bestimmt. Hiernach wird zumindest ein Parameter der trainierten Funktion basierend auf einem Vergleich des Bewertungsparameters mit einem Referenzwert angepasst. Ferner wird die trainierte Funktion bereitgestellt.

Die vorstehend beschriebenen Schritte des vorgeschlagenen Verfahrens können teilweise oder vollständig computerimplementiert sein. Zudem können die vorstehend beschriebenen Schritte des vorgeschlagenen Verfahrens zumindest teilweise, insbesondere vollständig, nacheinander oder zumindest teilweise gleichzeitig ausgeführt werden.

Die Trainingsprojektionsabbildungen können vorteilhafterweise alle Merkmale und Eigenschaften der Projektionsabbildungen aufweisen, welche in Bezug zu dem Verfahren zum Bereitstellen eines 3D-Ergebnisdatensatzes beschrieben wurden und umgekehrt. Das Erfassen der Trainingsprojektionsabbildungen kann ein Empfangen und/oder Aufnehmen der Projektionsabbildungen umfassen. Das Empfangen der Trainingsprojektionsabbildungen kann insbesondere ein Erfassen und/oder Auslesen eines computerlesbaren Datenspeichers und/oder ein Empfangen aus einer Datenspeichereinheit, beispielsweise einer Datenbank, umfassen, beispielsweise mittels einer Schnittstelle.

Gemäß einer ersten Variante können die Trainingsprojektionsabbildungen mittels des medizinischen Trainingsröntgengeräts, insbesondere einem medizinischen C-Bogen-Röntgengerät, aus den verschiedenen Projektionsrichtungen, insbesondere entlang der 3D-Trajektorie, aufgenommen werden. Die verschiedenen Projektionsrichtungen können vorteilhafterweise zumindest teilweise nicht kollinear sein.

Die Projektionsrichtungen können jeweils einen Verlauf eines Strahls, insbesondere eines Zentral- und/oder Mittenstrahls, zwischen einer Röntgenquelle und einem Röntgendetektor, insbesondere einem Detektormittelpunkt, des Trainingsröntgengeräts zum Aufnahmezeitpunkt der jeweiligen Trainingsprojektionsabbildungen beschreiben. Insbesondere können die Projektionsrichtungen jeweils eine Angulation des Trainingsröntgengeräts bezüglich des Untersuchungsobjekts und/oder eines Isozentrums, insbesondere Drehzentrums, einer definierten Anordnung von Röntgenquelle und Röntgendetektor beschreiben. Dabei kann das Isozentrum einen räumlichen Punkt beschreiben, um welchen die definierte Anordnung von Röntgenquelle und Röntgendetektor bewegbar, insbesondere rotierbar, ist, insbesondere während der Aufnahme der Trainingsprojektionsabbildungen. Vorteilhafterweise können die verschiedenen Projektionsrichtungen jeweils durch das, insbesondere gemeinsame, Isozentrum verlaufen. Die 3D-Trajektorie kann einen räumlichen Pfad zur Anordnung eines Referenzpunkts, beispielsweise eines Fokuspunkts, der definierten Anordnung von Röntgenquelle und Röntgendetektor beschreiben. Vorteilhafterweise können die mehreren Trainingsprojektionsabbildungen das Trainingsuntersuchungsobjekt jeweils 2D räumlich aufgelöst abbilden.

Alternativ können die Trainingsprojektionsabbildungen simuliert sein, beispielsweise mittels einer virtuellen Repräsentation des Untersuchungsobjekts und einem physikalischen Abbildungsmodell des Trainingsröntgengeräts.

Das Trainingsuntersuchungsobjekt kann alle Merkmale und Eigenschaften des Untersuchungsobjekts aufweisen, welche in Bezug zu dem Verfahren zum Bereitstellen eines 3D-Ergebnisdatensatzes beschrieben wurden und umgekehrt. Das Trainingsuntersuchungsobjekt kann gleich oder verschieden von dem Untersuchungsobjekt sein. Insbesondere kann das Verfahren zum Bereitstellen einer trainierten Funktion für verschiedene Trainingsuntersuchungsobjekte wiederholt ausgeführt werden.

Das Bereitstellen der initialen Trainingsprojektionsmatrix basierend auf dem statischen Trainingsmodell des Trainingsröntgengeräts kann analog zum Bereitstellen der initialen Projektionsmatrix erfolgen. Die initiale Trainingsprojektionsmatrix, das statische Trainingsmodell und das medizinische Trainingsröntgengerät können jeweils insbesondere alle Merkmale und Eigenschaften der initialen Projektionsmatrix, des statischen Modells und des medizinischen Röntgengeräts aufweisen, welche in Bezug zu dem Verfahren zum Bereitstellen eines 3D-Ergebnisdatensatzes beschrieben wurden und umgekehrt.

Die weitere Trainingsprojektionsmatrix kann durch das Anwenden der trainierten Funktion auf die Eingabedaten bereitgestellt werden. Dabei basieren die Eingabedaten der trainierten Funktion auf den Trainingsprojektionsabbildungen und der initialen Projektionsmatrix. Insbesondere können die Eingabedaten der trainierten Funktion die Trainingsprojektionsabbildungen und die initiale Projektionsmatrix umfassen.

Vorteilhafterweise kann der 3D-Trainingsdatensatz aus den mehreren Trainingsprojektionsabbildungen mittels der weiteren Trainingsprojektionsmatrix rekonstruiert werden, beispielsweise mittels einer gefilterten Rückprojektion.

Durch das Anwenden der Bildqualitätsmetrik und/oder der Konsistenzmetrik auf den 3D-Trainingsdatensatz kann vorteilhafterweise ein Bewertungsparameter, insbesondere ein Bildqualitätsparameter und/oder ein Konsistenzparameter, bereitgestellt werden. Der Bewertungsparameter kann die Bildqualität und/oder Konsistenz des 3D-Trainingsdatensatzes bewerten, insbesondere qualitativ und/oder quantitativ.

Vorteilhafterweise kann der zumindest eine Parameter der trainierten Funktion basierend auf dem Vergleich des Bewertungsparameters mit dem vorgegebenen Referenzwert derart angepasst werden, dass bei wiederholter Anwendung der trainierten Funktion auf die Eingabedaten und Rekonstruktion des 3D-Trainingsdatensatzes, der dabei bereitstellbare 3D-Trainingsdatensatz eine höhere Bildqualität und/oder Konsistenz gegenüber jeweils zuvor bereitgestellten 3D-Trainingsdatensatz aufweist. Steigt ein Wert des Bewertungsparameters monoton mit steigender Bildqualität und/oder Konsistenz, so kann der zumindest eine Parameter der trainierten Funktion vorteilhafterweise derart angepasst werden, dass der Bewertungsparameter des 3D-Trainingsdatensatzes bei wiederholter Anwendung der trainierten Funktion auf die Eingabedaten und Rekonstruktion des 3D-Traindatensatzes steigt. Sinkt ein Wert des Bewertungsparameters monoton mit steigender Bildqualität und/oder Konsistenz, so kann der zumindest eine Parameter der trainierten Funktion vorteilhafterweise derart angepasst werden, dass der Bewertungsparameter des 3D-Trainingsdatensatzes bei wiederholter Anwendung der trainierten Funktion auf die Eingabedaten und Rekonstruktion des 3D-Traindatensatzes sinkt.

Der Referenzwert kann, insbesondere anhand einer mittels einer Eingabeeinheit erfassten Nutzereingabe, empfangen werden. Alternativ oder zusätzlich kann der Referenzwert bestimmt werden. Der Referenzwert kann einen Schwellwert für eine Mindestbildqualität und/oder eine Mindestkonsistenz des 3D-Trainingsdatensatzes vorgeben. Vorteilhafterweise können das Bereitstellen der weiteren Trainingsprojektionsmatrix, das Rekonstruieren des 3D-Trainingsdatensatzes, das Bestimmen des Bewertungsparameters und das Anpassen des zumindest einen Parameters der trainierten Funktion so lange wiederholt ausgeführt werden, bis der Bewertungsparameter den Referenzwert erreicht oder übersteigt.

Das Bereitstellen der trainierten Funktion kann insbesondere ein Speichern auf einem computerlesbaren Speichermedium und/ oder ein Übertragen an eine Bereitstellungseinheit umfassen. Vorteilhafterweise kann mit dem vorgeschlagenen Verfahren eine trainierte Funktion bereitgestellt werden, welche in einer Ausführungsform des Verfahrens zum Bereitstellen eines 3D-Ergebnisdatensatzes verwendet werden kann.

Vorteilhafterweise kann mit dem vorgeschlagenen Verfahren eine trainierte Funktion bereitgestellt werden, welche in einer Ausführungsform des Verfahrens zum Bereitstellen eines 3D-Ergebnisdatensatzes verwendet werden kann. Vorteilhafterweise können Kriterien für eine Leistungsfähigkeit (engl. performance) der trainierten Funktion unabhängig von dedizierten Qualitätsmetriken sein, welche nur für dedizierte Bildinhalte anwendbar sind, sondern können allgemein anhand der Trainingsabbildungspaare gelernt und der trainierten Funktion überlassen werden.

In einer weiteren vorteilhaften Ausführungsform des vorgeschlagenen Verfahrens zum Bereitstellen einer trainierten Funktion kann ein 3D-Vergleichsdatensatz aus den Trainingsprojektionsabbildungen mittels der initialen Trainingsprojektionsmatrix rekonstruiert werden. Ferner kann der Referenzwert durch Anwenden der Bildqualitätsmetrik und/oder der Konsistenzmetrik auf den 3D-Vergleichsdatensatz bestimmt werden.

Vorteilhafterweise kann der 3D-Vergleichsdatensatz aus den mehreren Trainingsprojektionsabbildungen mittels der initialen Trainingsprojektionsmatrix rekonstruiert werden, beispielsweise mittels einer gefilterten Rückprojektion.

Durch das Anwenden der Bildqualitätsmetrik und/oder der Konsistenzmetrik auf den 3D-Vergleichsdatensatz kann vorteilhafterweise der Referenzwert, insbesondere aufweisend einen Bildqualitätsparameter und/oder einen Konsistenzparameter, bereitgestellt werden. Der Referenzwert kann die Bildqualität und/oder Konsistenz des 3D-Vergleichsdatensatzes bewerten, insbesondere qualitativ und/oder quantitativ.

Vorteilhafterweise kann anhand des Vergleichs des Bewertungsparameters mit dem Referenzwert identifiziert werden, ob der 3D-Vergleichsdatensatz eine höhere Bildqualität und/oder Konsistenz gegenüber dem 3D-Trainingsdatensatz aufweist. Ferner kann der zumindest eine Parameter der trainierten Funktion vorteilhaft derart angepasst werden, dass bei wiederholter Anwendung der trainierten Funktion auf die Eingabedaten und Rekonstruktion des 3D-Trainingsdatensatzes, der dabei bereitstellbare 3D-Trainingsdatensatz eine höhere Bildqualität und/oder Konsistenz gegenüber dem 3D-Vergleichsdatensatz aufweist.

In einer weiteren vorteilhaften Ausführungsform des vorgeschlagenen Verfahrens zum Bereitstellen einer trainierten Funktion können jeweils eine initiale und eine weitere Trainingsprojektionsmatrix zu den verschiedenen Projektionsrichtungen bereitgestellt werden. Zudem können die Eingabedaten der trainierten Funktion auf den initialen Trainingsprojektionsmatrizen basieren. Ferner kann der 3D-Trainingsdatensatz durch Rekonstruktion aus den Trainingsprojektionsabbildungen mittels der weiteren Trainingsprojektionsmatrizen bereitgestellt werden.

Vorteilhafterweise kann zu jeder verschiedenen Projektionsrichtung der mehreren Projektionsrichtungen jeweils eine initiale Trainingsprojektionsmatrix basierend auf dem statischen Trainingsmodell des Trainingsröntgengeräts bereitgestellt werden. Vorteilhafterweise kann der 3D-Trainingsdatensatz aus den mehreren Projektionsabbildungen rekonstruiert werden, wobei die mit der Projektionsrichtung der jeweiligen Trainingsprojektionsabbildung korrespondierende initiale Trainingsprojektionsmatrix verwendet wird. Die Eingabedaten der trainierten Funktion können vorteilhafterweise auf den mehreren initialen Trainingsprojektionsmatrizen basieren, insbesondere die mehreren initialen Trainingsprojektionsmatrizen umfassen. Ferner kann die trainierte Funktion zu jeder verschiedenen Projektionsrichtung der mehreren Projektionsrichtungen jeweils eine weitere Trainingsprojektionsmatrix als Ausgabedaten bereitstellen.

Sofern ein 3D-Vergleichsdatensatz rekonstruiert wird, kann der 3D-Vergleichsdatensatz aus den mehreren Trainingsprojektionsabbildungen rekonstruiert werden, wobei die mit der Projektionsrichtung der jeweiligen Projektionsabbildung korrespondierende weitere Trainingsprojektionsmatrix verwendet wird.

Mittels der vorgeschlagenen Ausführungsform kann die trainierte Funktion vorteilhaft zum Bereitstellen dedizierter weiterer Trainingsprojektionsmatrizen zu den verschiedenen Projektionsrichtungen ausgebildet werden.

In einer weiteren vorteilhaften Ausführungsform des vorgeschlagenen Verfahrens zum Bereitstellen einer trainierten Funktion kann eine Information zu dynamischen Bewegungsfreiheitsgraden des Trainingsröntgengeräts erfasst werden, welche einen latenten Trainingsraum definiert. Dabei können die Eingabedaten der trainierten Funktion zusätzlich auf dem latenten Trainingsraum basieren.

Das medizinische Trainingsröntgengerät kann mehrere dynamische Bewegungsfreiheitsgrade aufweisen. Die dynamischen Bewegungsfreiheitsgrade können beispielsweise eine Wirkung von Reibung und/oder Dämpfung und/oder Schwingungen und/oder Trägheitsmomenten und/oder Zentrifugaleffekten und/oder Corioliseffekten auf eine Bewegung des Trainingsröntgengeräts kennzeichnen. Insbesondere können die dynamischen Bewegungsfreiheitsgrade einen maximalen Einfluss von Reibung und/oder Dämpfung und/oder Schwingungen und/oder Trägheitsmomenten und/oder Zentrifugaleffekten und/oder Corioliseffekten auf die Bewegung des Trainingsröntgengeräts beschreiben. Der latente Trainingsraum kann durch die dynamischen Bewegungsfreiheitsgrade, insbesondere auch durch statische Bewegungsfreiheitsgrade, des Trainingsröntgengeräts definiert, insbesondere vorgegeben und/oder begrenzt, werden. Die dynamischen Bewegungsfreiheitsgrade, insbesondere der latente Trainingsraum, können physikalisch mögliche dynamische Abweichungen von statischen Bewegungen des Trainingsröntgengeräts beschreiben. Der latente Trainingsraum kann vorteilhafterweise alle Merkmale und Eigenschaften des latenten Raums aufweisen, welche in Bezug zu dem Verfahren zum Bereitstellen eines 3D-Ergebnisdatensatzes beschrieben wurden und umgekehrt.

Die dynamischen Bewegungsfreiheitsgrade, insbesondere der latente Trainingsraum, des Röntgengeräts können gelernt sein, beispielsweise mittels eines Variationsautoencoders. Die dynamischen Bewegungsfreiheitsgrade, insbesondere der latente Trainingsraum, können vorteilhafterweise anhand von Abweichungen zwischen initialen Trainingsprojektionsmatrizen, welche anhand des statischen Trainingsmodells des Trainingsröntgengeräts bestimmt sind, und Kalibrierprojektionsmatrizen, welche anhand von Kalibrierprojektionsabbildungen eines Phantoms bekannter Geometrie bestimmt sind, ermittelt werden.

Vorteilhafterweise können die Eingabedaten der trainierten Funktion zusätzlich den latenten Trainingsraum, insbesondere eine den latenten Trainingsraum charakterisierende Information, umfassen.

Mittels der vorgeschlagenen Ausführungsform kann die trainierte Funktion vorteilhaft zum Bereitstellen der wenigstens einen Trainingsprojektionsmatrix unter Berücksichtigung der dynamischen Bewegungsfreiheitsgrade des Trainingsröntgengeräts ausgebildet werden.

In einer weiteren vorteilhaften Ausführungsform des vorgeschlagenen Verfahrens zum Bereitstellen einer trainierten Funktion können die Trainingsprojektionsabbildungen für die verschiedenen Projektionsrichtungen basierend auf dem statischen Modell und dem latenten Trainingsraum simuliert werden.

Realistische dynamische Abweichungen vom statischen Modell können beispielsweise durch Messungen mit einem Kalibrierphantom an dem Trainingsröntgengerät bestimmt werden. Hierbei können Messdaten erfasst werden. Mittels der Messdaten als Eingabedaten kann ein neuronales Autoencoder Netzwerk trainiert werden, welches den latenten Trainingsraum der dynamischen Abweichungen beschreibt. Durch eine zufällige Modulation von Eingangsdaten eines Decoders des Autoencoder Netzwerks können beliebige weitere realistische dynamische Abweichungen erzeugt werden. Die gemessenen und/oder erzeugten dynamischen Abweichungen können mit dem statischen Modell kombiniert werden, um ein realistisches dynamisches Modell, insbesondere den latenten Trainingsraum, des Trainingsröntgengeräts zu erzeugen. Mittels virtueller, insbesondere digitaler, Vorwärtsprojektion von klinischen 3D-Bilddaten eines Trainingsuntersuchungsobjekts können realistische Trainingsprojektionsabbildungen simuliert werden.

In einer weiteren vorteilhaften Ausführungsform des vorgeschlagenen Verfahrens zum Bereitstellen einer trainierten Funktion kann die Konsistenzmetrik eine epipolare Konsistenz und/oder eine Konsistenz von Vorwärtsprojektionen des 3D-Trainingsdatensatzes mit den Trainingsprojektionsabbildungen bewerten. Alternativ oder zusätzlich kann die Bildqualitätsmetrik eine Gesamtvariation des 3D-Vergleichsdatensatzes und des 3D-Trainingsdatensatzes bewerten.

Die epipolare Konsistenzbedingung kann auf Redundanzen in den aufgenommenen Trainingsprojektionsabbildungen im Radonraum basieren, welche über Geometrieparameter der verschiedenen Projektionsrichtungen, insbesondere der 3D-Trajektorie, bestimmt werden. Passt die angenommene 3D-Trajektorie aufgrund von dynamischen Abweichungen nicht zu den Trainingsprojektionsabbildungen, können diese Abweichungen über eine entsprechende Konsistenzmetrik detektiert werden. Eine derartige Detektion ist beispielsweise aus der Druckschrift von Robert Frysch und Georg Rose, "Rigid motion compensation in interventional C-arm CT using consistency measure on projection data", Medical Image Computing and Computer-Assisted Intervention--MICCAI 2015: 18th International Conference, Munich, Germany, October 5-9, 2015, Proceedings, Part I, 18., Springer International Publishing, bekannt. Dabei kann die Konsistenzmetrik einen Bewertungsparameter für den 3D-Trainingsdatensatz bereitstellen, wobei ein Wert des Bewertungsparameters die epipolare Konsistenz des 3D-Trainingsdatensatzes charakterisiert, insbesondere quantifiziert.

Vorteilhafterweise können durch virtuelle Vorwärtsprojektion zu den Trainingsprojektionsabbildungen, insbesondere den Projektionsrichtungen der Trainingsprojektionsabbildungen, jeweils virtuelle Projektionsabbildungen des 3D-Trainingsdatensatzes bestimmt werden. Die Konsistenzmetrik kann vorteilhafterweise eine Konsistenz, insbesondere eine Übereinstimmung, der virtuellen Vorwärtsprojektionen, insbesondere der virtuellen Projektionsabbildungen, des 3D-Trainingsdatensatzes mit den gemäß der Projektionsrichtung korrespondierenden Trainingsprojektionsabbildungen bewerten. Dabei kann die Konsistenzmetrik einen Bewertungsparameter für den 3D-Trainingsdatensatz bereitstellen, wobei ein Wert des Bewertungsparameters die Konsistenz der Vorwärtsprojektionen mit den Trainingsprojektionsabbildungen charakterisiert, insbesondere quantifiziert.

Die Bildqualitätsmetrik kann beispielsweise die Gesamtvariation (engl. total variation) von Bildwerten, beispielsweise Intensitätswerten und/oder Schwächungswerten, des 3D-Trainingsdatensatzes bewerten. Dabei kann die Bildqualitätsmetrik einen Bewertungsparameter für den 3D-Trainingsdatensatz bereitstellen, wobei ein Wert des Bewertungsparameters die Gesamtvariation charakterisiert, insbesondere quantifiziert.

In einer weiteren vorteilhaften Ausführungsform des vorgeschlagenen Verfahrens zum Bereitstellen einer trainierten Funktion können die Eingabedaten der trainierten Funktion zusätzlich auf dem statischen Trainingsmodell des Trainingsröntgengeräts basieren.

Vorteilhafterweise können die Eingabedaten der trainierten Funktion zusätzlich das statische Trainingsmodell des Trainingsröntgengeräts umfassen. Hierdurch kann die trainierte Funktion vorteilhaft dazu ausgebildet werden, die wenigstens eine weitere Projektionsmatrix zusätzlich unter Berücksichtigung des statischen Modells des Röntgengeräts bereitzustellen. Insbesondere kann eine robuste und akkurate Anpassung des wenigstens einen Parameters der trainierten Funktion sichergestellt werden, sodass die Ausgabedaten der trainierten Funktion mit hoher Genauigkeit und zeiteffizient bereitgestellt werden können. Das statische Trainingsmodell kann dabei als zusätzliche Eingabedaten zusätzliche Gewichte für die Bestimmung der zumindest einen weiteren Projektionsmatrix bereitstellen, da beispielsweise positionsabhängige direktionale mechanische Konformitätswerte zu Hauptrichtungen von dynamischen Effekten führen. Die Gewichte können für einzelne Projektionsrichtungen bereitgestellt werden, wodurch ein Parameterraum signifikant reduziert und eine robuste und schnelle Konvergenz der datenbasierten Optimierung sichergestellt werden kann.

Die Erfindung betrifft in einem dritten Aspekt eine Bereitstellungseinheit, welche zur Ausführung eines vorgeschlagenen Verfahrens zum Bereitstellen eines 3D-Ergebnisdatensatzes ausgebildet ist.

Dabei kann die Bereitstellungseinheit eine Recheneinheit, eine Speichereinheit und/oder eine Schnittstelle umfassen. Die Bereitstellungseinheit kann dazu ausgebildet sein, ein vorgeschlagenes Verfahren zum Bereitstellen eines 3D-Ergebnisdatensatzes auszuführen, in dem die Schnittstelle, die Recheneinheit und/oder die Speichereinheit dazu ausgebildet sind, die entsprechenden Verfahrensschritte auszuführen.

Vorteilhafterweise kann die Schnittstelle dazu ausgebildet sein, die Projektionsabbildungen zu erfassen und/oder den 3D-Ergebnisdatensatz bereitzustellen. Ferner können die Recheneinheit und/oder die Speichereinheit dazu ausgebildet sein, die initiale und die weitere Trainingsprojektionsmatrix bereitzustellen.

Die Vorteile der vorgeschlagenen Bereitstellungseinheit entsprechen im Wesentlichen den Vorteilen des vorgeschlagenen Verfahrens zum Bereitstellen eines 3D-Ergebnisdatensatzes. Hierbei erwähnte Merkmale, Vorteile oder alternative Ausführungsformen können ebenso auch auf die anderen beanspruchten Gegenstände übertragen werden und umgekehrt.

Die Erfindung betrifft in einem vierten Aspekt ein medizinisches Röntgengerät, umfassend eine vorgeschlagene Bereitstellungseinheit. Das Röntgengerät ist dazu ausgebildet, die Projektionsabbildungen des Untersuchungsobjekts, insbesondere entlang der 3D-Trajektorie, aus den verschiedenen Projektionsrichtungen aufzunehmen.

Die Vorteile des vorgeschlagenen Röntgengeräts entsprechen im Wesentlichen den Vorteilen des vorgeschlagenen Verfahrens zum Bereitstellen eines 3D-Ergebnisdatensatzes. Hierbei erwähnte Merkmale, Vorteile oder alternative Ausführungsformen können ebenso auch auf die anderen beanspruchten Gegenstände übertragen werden und umgekehrt.

Die Erfindung betrifft in einem fünften Aspekt eine Trainingseinheit, welche dazu ausgebildet ist, ein vorgeschlagenes Verfahren zum Bereitstellen einer trainierten Funktion auszuführen. Dabei kann die Trainingseinheit vorteilhafterweise eine Trainingsschnittstelle, eine Trainingsspeichereinheit und/oder eine Trainingsrecheneinheit umfassen. Die Trainingseinheit kann dazu ausgebildet sein, ein Verfahren zum Bereitstellen einer trainierten Funktion auszuführen, indem die Trainingsschnittstelle, die Trainingsspeichereinheit und/oder die Trainingsrecheneinheit dazu ausgebildet sind, die entsprechenden Verfahrensschritte auszuführen.

Vorteilhafterweise kann die Schnittstelle dazu ausgebildet sein, die Trainingsprojektionsabbildungen zu erfassen und/oder die trainierte Funktion bereitzustellen. Ferner können die Recheneinheit und/oder die Speichereinheit dazu ausgebildet sein, die initiale und die weitere Trainingsprojektionsmatrix bereitzustellen, den 3D-Vergleichsdatensatz und den 3D-Trainingsdatensatz zu rekonstruieren, die Bewertungsparameter zu bestimmen und den zumindest einen Parameter der trainierten Funktion anzupassen.

Die Vorteile der vorgeschlagenen Trainingseinheit entsprechen im Wesentlichen den Vorteilen des vorgeschlagenen Verfahrens zum Bereitstellen einer trainierten Funktion. Hierbei erwähnte Merkmale, Vorteile oder alternative Ausführungsformen können ebenso auch auf die anderen beanspruchten Gegenstände übertragen werden und umgekehrt.

Die Erfindung betrifft in einem sechsten Aspekt ein Computerprogrammprodukt mit einem Computerprogramm, welches direkt in einen Speicher einer Bereitstellungseinheit ladbar ist, mit Programmabschnitten, um alle Schritte des Verfahrens zum Bereitstellen eines 3D-Ergebnisdatensatzes und/oder eines seiner Aspekte auszuführen, wenn die Programmabschnitte von der Bereitstellungseinheit ausgeführt werden; und/oder welches direkt in einen Trainingsspeicher einer Trainingseinheit ladbar ist, mit Programmabschnitten, um alle Schritte eines vorgeschlagenen Verfahrens zum Bereitstellen einer trainierten Funktion und/oder eines seiner Aspekte auszuführen, wenn die Programmabschnitte von der Trainingseinheit ausgeführt werden.

Die Erfindung kann ferner ein Computerprogramm oder computerlesbares Speichermedium betreffen, umfassend eine trainierte Funktion, welche durch ein vorgeschlagenes Verfahren oder eines seiner Aspekte bereitgestellt wurde.

Eine weitgehend softwaremäßige Realisierung hat den Vorteil, dass schon bisher verwendete Bereitstellungseinheiten und/ oder Trainingseinheiten auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie zum Beispiel eine Dokumentation und/oder zusätzliche Komponenten, sowie Hardware-Komponenten, wie zum Beispiel Hardware-Schlüssel (Dongles etc.) zur Nutzung der Software, umfassen.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet. Es zeigen:
Fig. 1 und 2 schematische Darstellungen vorteilhafter Ausführungsformen eines vorgeschlagenen Verfahrens zum Bereitstellen eines 3D-Ergebnisdatensatzes,
Fig. 3 bis 5 schematische Darstellungen vorteilhafter Ausführungsformen eines vorgeschlagenen Verfahrens zum Bereitstellen einer trainierten Funktion,
Fig. 6 eine schematische Darstellung einer vorgeschlagenen Bereitstellungseinheit,
Fig. 7 eine schematische Darstellung einer vorgeschlagenen Trainingseinheit,
Fig. 8 eine schematische Darstellung eines vorgeschlagenen medizinischen Röntgengeräts.

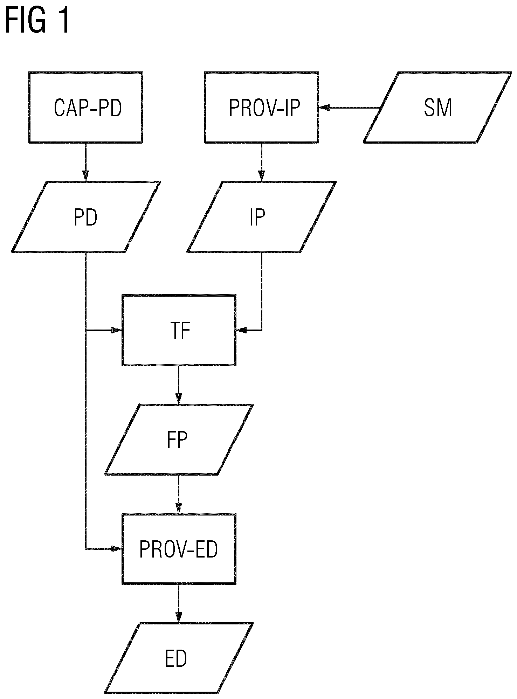

Fig. 1 zeigt eine schematische Darstellung einer vorteilhaften Ausführungsform eines vorgeschlagenen Verfahrens zum Bereitstellen eines 3D-Ergebnisdatensatzes PROV-ED. Dabei können Projektionsabbildungen PD eines Untersuchungsobjekts erfasst werden, welche mittels eines medizinischen Röntgengeräts, insbesondere entlang einer 3D-Trajektorie, aus verschiedenen Projektionsrichtungen aufgenommen sind. Ferner kann eine initiale Projektionsmatrix IP, insbesondere jeweils eine initiale Projektionsmatrix IP zu den verschiedenen Projektionsrichtungen, basierend auf einem statischen Modell SM des Röntgengeräts bereitgestellt werden PROV-IP. Zudem kann eine weitere Projektionsmatrix FP, insbesondere jeweils eine weitere Projektionsmatrix FP zu den verschiedenen Projektionsrichtungen, durch Anwenden einer trainierten Funktion TF auf Eingabedaten bereitgestellt werden. Dabei können die Eingabedaten der trainierten Funktion TF auf der initialen Projektionsmatrix IP, insbesondere den initialen Projektionsmatrizen IP, und den Projektionsabbildungen PD basieren. Zudem kann zumindest ein Parameter der trainierten Funktion TF basierend auf einer Bildqualitätsmetrik und/oder einer Konsistenzmetrik angepasst sein. Die weitere Projektionsmatrix FP, insbesondere die weiteren Projektionsmatrizen, kann als Ausgabedaten der trainierten Funktion TF bereitgestellt werden. Hiernach kann der 3D-Ergebnisdatensatz ED durch Rekonstruktion aus den Projektionsabbildungen PD mittels der weiteren Projektionsmatrix FP, insbesondere den weiteren Projektionsmatrizen FP, bereitgestellt werden PROV-ED.

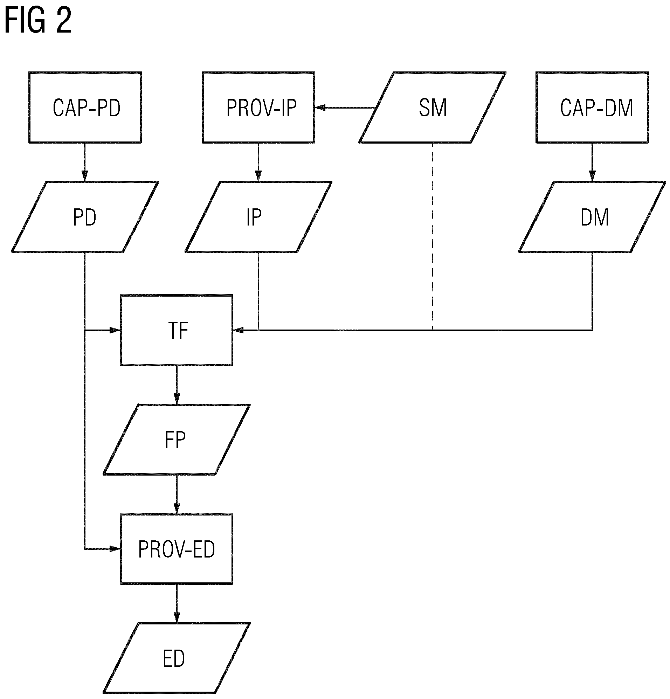

Fig. 2 zeigt eine schematische Darstellung einer weiteren vorteilhaften Ausführungsform des vorgeschlagenen Verfahrens zum Bereitstellen eines 3D-Ergebnisdatensatzes PROV-ED. Dabei können die Eingabedaten der trainierten Funktion TF zusätzlich auf dem statischen Modell SM des Röntgengeräts basieren. Vorteilhafterweise kann ferner eine Information zu dynamischen Bewegungsfreiheitsgraden des Röntgengeräts erfasst werden CAP-DM, welche einen latenten Raum DM definiert. Dabei können die Eingabedaten der trainierten Funktion TF zusätzlich auf dem latenten Raum DM basieren.

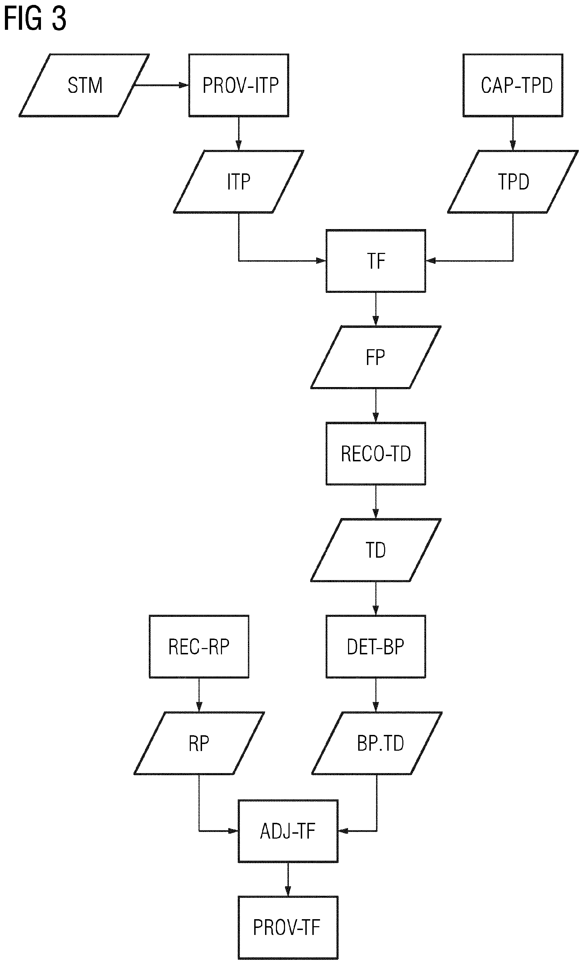

In Fig. 3 ist eine vorteilhafte Ausführungsform einer vorteilhaften Ausführungsform eines vorgeschlagenen Verfahrens zum Bereitstellen einer trainierten Funktion PROV-TF schematisch dargestellt. In einem ersten Schritt können Trainingsprojektionsabbildungen TPD eines Trainingsuntersuchungsobjekts erfasst werden CAP-TPD, welche das Trainingsuntersuchungsobjekt, insbesondere entlang einer 3D-Trajektorie, aus verschiedenen Projektionsrichtungen abbilden. Die Trainingsprojektionsabbildungen TPD können mittels des Trainingsröntgengeräts, insbesondere entlang der 3D-Trajektorie, aus den verschiedenen Projektionsrichtungen aufgenommen werden. Ferner kann eine initiale Trainingsprojektionsmatrix ITP basierend auf einem statischen Trainingsmodell TM eines medizinischen Trainingsröntgengeräts bereitgestellt werden PROV-ITP. Insbesondere kann jeweils eine initiale Trainingsprojektionsmatrix ITP zu den verschiedenen Projektionsrichtungen bereitgestellt werden. Ferner kann eine weitere Trainingsprojektionsmatrix IFP, insbesondere jeweils eine weitere Trainingsprojektionsmatrix IFP zu den verschiedenen Projektionsrichtungen, durch Anwenden der trainierten Funktion TF auf Eingabedaten bereitgestellt werden. Dabei können die Eingabedaten der trainierten Funktion TF auf den Trainingsprojektionsabbildungen TPD und der initialen Trainingsprojektionsmatrix ITP, insbesondere den mehreren initialen Trainingsprojektionsmatrizen ITP, basieren. Ferner kann die weitere Trainingsprojektionsmatrix IFP, insbesondere die mehreren weiteren Trainingsprojektionsmatrizen IFP, als Ausgabedaten der trainierten Funktion TF bereitgestellt werden. Vorteilhafterweise kann ein 3D-Trainingsdatensatz TD aus den Trainingsprojektionsabbildungen TPD mittels der weiteren Trainingsprojektionsmatrix IFP rekonstruiert werden. Ferner kann jeweils ein Bewertungsparameter BP.VD und BP.TD durch Anwenden einer Bildqualitätsmetrik und/oder einer Konsistenzmetrik auf den 3D-Trainingsdatensatz TD bestimmt werden DET-BP. Die Konsistenzmetrik kann eine epipolare Konsistenz und/oder eine Konsistenz des 3D-Trainingsdatensatzes TD mit den Trainingsprojektionsabbildungen TPD bewerten. Alternativ oder zusätzlich kann die Bildqualitätsmetrik eine Gesamtvariation des 3D-Trainingsdatensatzes TD bewerten. Hiernach kann zumindest ein Parameter der trainierten Funktion TF basierend auf einem Vergleich des Bewertungsparameters BP.TD mit einem Referenzwert RP derart angepasst werden ADJ-TF. Der Referenzwert RP kann empfangen werden, beispielsweise anhand einer Nutzereingabe, und/oder bestimmt werden REC-RP. Zudem kann die trainierte Funktion TF bereitgestellt werden PROV-TF.

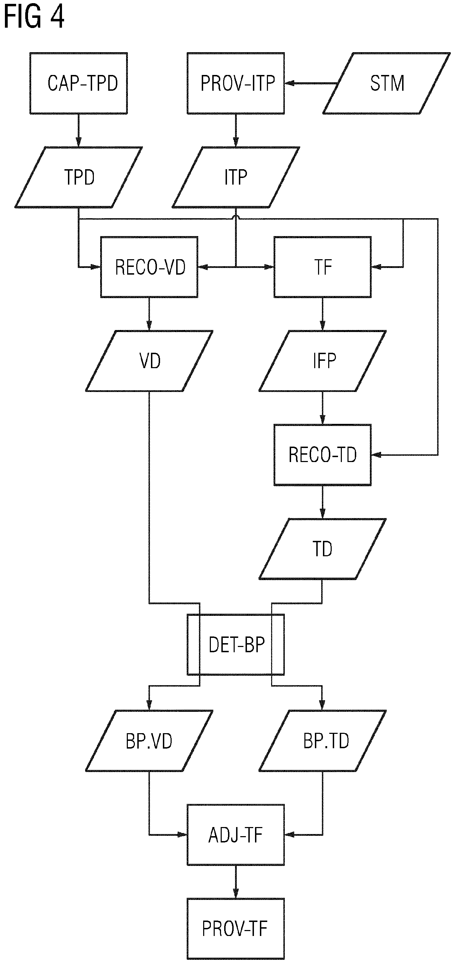

In Fig. 4 ist eine vorteilhafte Ausführungsform einer weiteren vorteilhaften Ausführungsform des vorgeschlagenen Verfahrens zum Bereitstellen einer trainierten Funktion PROV-TF schematisch dargestellt. Dabei kann ein 3D-Vergleichsdatensatz VD aus den Trainingsprojektionsabbildungen TPD mittels der initialen Trainingsprojektionsmatrix ITP rekonstruiert werden RECO-VD. Ferner kann der Referenzwert RP als Bewertungsparameter BP.VD durch Anwenden DET-BP der Bildqualitätsmetrik und/oder der Konsistenzmetrik auf den 3D-Vergleichsdatensatz VD bestimmt werden.

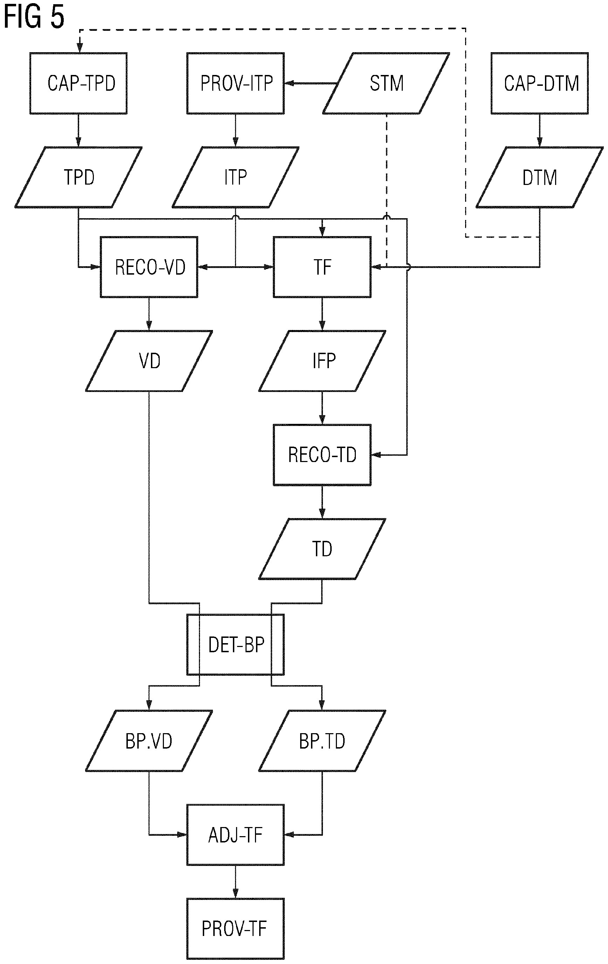

In Fig. 5 ist eine vorteilhafte Ausführungsform einer weiteren vorteilhaften Ausführungsform des vorgeschlagenen Verfahrens zum Bereitstellen einer trainierten Funktion PROV-TF schematisch dargestellt. Dabei kann eine Information zu dynamischen Bewegungsfreiheitsgraden des Trainingsröntgengeräts erfasst werden CAP-DTM, welche einen latenten Trainingsraum DTM definiert. Dabei können die Eingabedaten der trainierten Funktion TF zusätzlich auf dem latenten Trainingsraum DTM basieren. Zudem können die Trainingsprojektionsabbildungen TPD für die verschiedenen Projektionsrichtungen basierend auf dem statischen Trainingsmodell STM und dem latenten Raum DTM simuliert werden. Die Eingabedaten der trainierten Funktion TF können zusätzlich auf dem statischen Trainingsmodell STM des Trainingsröntgengeräts basieren.

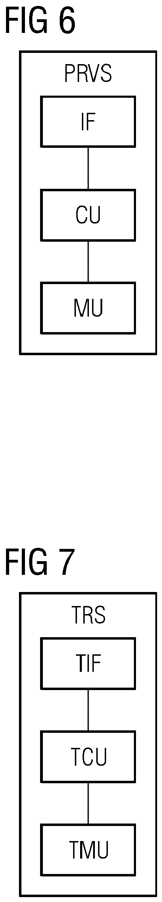

Fig. 6 zeigt eine schematische Darstellung einer vorgeschlagenen Bereitstellungseinheit PRVS. Dabei kann die Bereitstellungseinheit PRVS eine Recheneinheit CU, eine Speichereinheit MU und/oder eine Schnittstelle IF umfassen. Die Bereitstellungseinheit PRVS kann dazu ausgebildet sein, ein vorgeschlagenes Verfahren zum Bereitstellen eines 3D-Ergebnisdatensatzes PROV-ED auszuführen, in dem die Schnittstelle IF, die Recheneinheit CU und/oder die Speichereinheit MU dazu ausgebildet sind, die entsprechenden Verfahrensschritte auszuführen. Vorteilhafterweise kann die Schnittstelle IF dazu ausgebildet sein, die Projektionsabbildungen PD zu erfassen und/oder den 3D-Ergebnisdatensatz 3D bereitzustellen. Ferner können die Recheneinheit CU und/oder die Speichereinheit MU dazu ausgebildet sein, die initiale und die weitere Trainingsprojektionsmatrix IP und FP bereitzustellen.

Fig. 7 zeigt eine schematische Darstellung einer vorgeschlagenen Trainingseinheit TRS. Dabei kann die Trainingseinheit TRS vorteilhafterweise eine Trainingsschnittstelle TIF, eine Trainingsspeichereinheit TMU und/oder eine Trainingsrecheneinheit TCU umfassen. Die Trainingseinheit TRS kann dazu ausgebildet sein, ein Verfahren zum Bereitstellen einer trainierten Funktion PROV-TF auszuführen, indem die Trainingsschnittstelle TIF, die Trainingsspeichereinheit TMU und/oder die Trainingsrecheneinheit TCU dazu ausgebildet sind, die entsprechenden Verfahrensschritte auszuführen. Vorteilhafterweise kann die Schnittstelle IF dazu ausgebildet sein, die Trainingsprojektionsabbildungen TPD zu erfassen und/oder die trainierte Funktion TF bereitzustellen PROV-TF. Ferner können die Recheneinheit CU und/oder die Speichereinheit MU dazu ausgebildet sein, die initiale und die weitere Trainingsprojektionsmatrix ITP und IFP bereitzustellen, den 3D-Trainingsdatensatz TD zu rekonstruieren, den Bewertungsparameter BP.TD zu bestimmen und den zumindest einen Parameter der trainierten Funktion TF anzupassen ADJ-TF. Die Trainingsschnittstelle IF kann zum Empfangen des Referenzwerts RP ausgebildet sein. Alternativ können die Trainingsrecheneinheit TCU und/oder die Trainingsspeichereinheit TMU zum Bestimmen des Referenzwerts RP als Bewertungsparameter BP.VD durch das Anwenden DET-BP der Bildqualitätsmetrik und/oder Konsistenzmetrik auf den 3D-Vergleichsdatensatz VD ausgebildet sein.

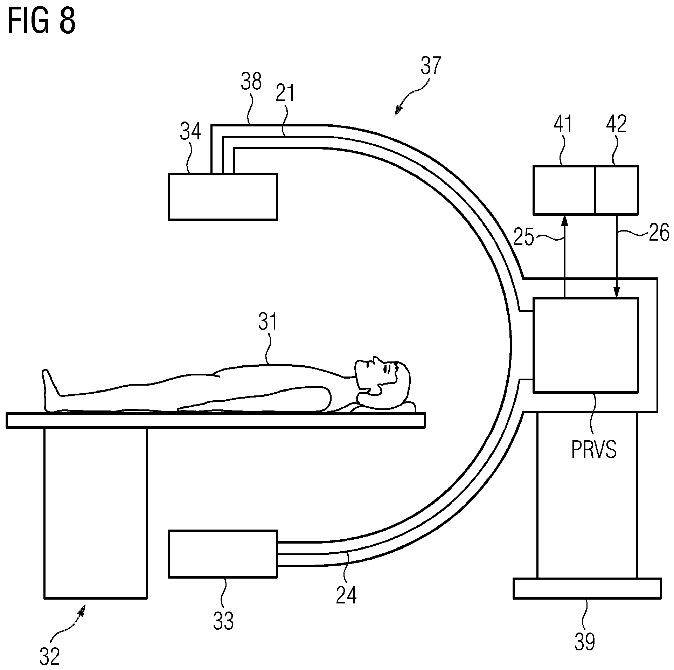

Fig. 8 zeigt beispielhaft für ein medizinisches Röntgengerät eine schematische Darstellung eines medizinischen C-Bogen-Röntgengeräts 37, umfassend eine vorgeschlagene Bereitstellungseinheit PRVS. Das medizinische C-Bogen-Röntgengerät 37 kann vorteilhafterweise einen Detektor 34, insbesondere einen Röntgendetektor, und eine Quelle 33, insbesondere eine Röntgenquelle, aufweisen, welche in definierter Anordnung an einem C-Arm 38 angeordnet sind. Der C-Arm 38 des C-Bogen-Röntgengeräts 37 kann beweglich um ein oder mehrere Achsen herum gelagert sein. Ferner kann das C-Bogen-Röntgengerät 37 eine Bewegungseinheit 39, beispielsweise ein Radsystem und/oder einen Roboterarm und/oder ein Schienensystem, umfassen, welche eine Bewegung des C-Bogen-Röntgengeräts im Raum ermöglicht. Zur Aufnahme der Projektionsabbildungen PD des, auf einer Patientenlagerungsvorrichtung 32 positionierten, Untersuchungsobjekts 31, kann die Bereitstellungseinheit PRVS ein Signal 24 an die Röntgenquelle 33 senden. Daraufhin kann die Röntgenquelle 33 ein Röntgenstrahlenbündel aussenden. Beim Auftreffen des Röntgenstrahlenbündels, nach einer Wechselwirkung mit dem Untersuchungsobjekt 31, auf einer Oberfläche des Detektors 34, kann der Detektor 34 ein Signal 21 an die Bereitstellungseinheit PRVS senden. Die Bereitstellungseinheit PRVS kann anhand des Signals 21 die Projektionsabbildungen PD erfassen.

Das Röntgengerät kann ferner eine Eingabeeinheit 42, beispielsweise eine Tastatur, und eine Darstellungseinheit 41, beispielsweise einen Monitor und/oder ein Display und/oder einen Projektor, aufweisen. Die Eingabeeinheit 42 kann vorzugsweise in die Darstellungseinheit 41 integriert sein, beispielsweise bei einem kapazitiven und/oder resistiven Eingabedisplay. Die Eingabeeinheit 42 kann vorteilhafterweise zur Erfassung einer Nutzereingabe ausgebildet sein. Hierfür kann die Eingabeeinheit 42 beispielsweise ein Signal 26 an die Bereitstellungseinheit PRVS senden. Die Bereitstellungseinheit PRVS kann dazu ausgebildet sein, die Aufnahme der Projektionsabbildungen PD anhand der Nutzereingabe zu steuern. Beispielsweise kann die 3D-Trajektorie anhand der Nutzereingabe vorgegeben werden.

Die Darstellungseinheit 41 kann vorteilhafterweise dazu ausgebildet sein, eine graphische Darstellung des 3D-Ergebnisdatensatzes anzuzeigen. Hierfür kann die Bereitstellungseinheit PRVS ein Signal 25 an die Darstellungseinheit 41 senden.

Die in den beschriebenen Figuren enthaltenen schematischen Darstellungen bilden keinerlei Maßstab oder Größenverhältnisse ab.

Es wird abschließend noch einmal darauf hingewiesen, dass es sich bei den vorhergehenden detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden Teil-Komponenten bestehen, die gegebenenfalls auch räumlich verteilt sein können.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

## Patentansprüche

1. Computerimplementiertes Verfahren zum Bereitstellen eines 3D-Ergebnisdatensatzes (PROV-ED), umfassend:
- Erfassen (CAP-PD) von Projektionsabbildungen (PD) eines Untersuchungsobjekts (31), welche mittels eines medizinischen Röntgengeräts aus verschiedenen Projektionsrichtungen aufgenommen sind,
- Bereitstellen (PROV-IP) einer initialen Projektionsmatrix (IP) basierend auf einem statischen Modell (SM) des Röntgengeräts,
- Bereitstellen einer weiteren Projektionsmatrix (FP) durch Anwenden einer trainierten Funktion (TF) auf Eingabedaten, wobei die Eingabedaten auf der initialen Projektionsmatrix (IP) und den Projektionsabbildungen (PD) basieren,
wobei zumindest ein Parameter der trainierten Funktion (TF) basierend auf einer Bildqualitätsmetrik und/oder einer Konsistenzmetrik angepasst ist,
wobei die weitere Projektionsmatrix (FP) als Ausgabedaten der trainierten Funktion (TF) bereitgestellt wird,
- Bereitstellen (PROV-ED) des 3D-Ergebnisdatensatzes (ED) durch Rekonstruktion aus den Projektionsabbildungen (PD) mittels der weiteren Projektionsmatrix (FP).

2. Verfahren nach Anspruch 1,
wobei jeweils eine initiale (IP) und eine weitere Projektionsmatrix (FP) zu den verschiedenen Projektionsrichtungen bereitgestellt werden (PROV-IP),
wobei die Eingabedaten der trainierten Funktion (TF) auf den initialen Projektionsmatrizen (IP) basieren,
wobei der 3D-Ergebnisdatensatz (ED) durch Rekonstruktion aus den Projektionsabbildungen (PD) mittels der weiteren Projektionsmatrizen (FP) bereitgestellt wird (PROV-ED).

3. Verfahren nach Anspruch 1 oder 2,
wobei die Eingabedaten der trainierten Funktion (TF) zusätzlich auf dem statischen Modell (SM) des Röntgengeräts basieren.

4. Verfahren nach einem der vorangehenden Ansprüche,
wobei eine Information zu dynamischen Bewegungsfreiheitsgraden des Röntgengeräts erfasst wird (CAP-DM), welche einen latenten Raum (DM) definiert,
wobei die Eingabedaten der trainierten Funktion (TF) zusätzlich auf dem latenten Raum (DM) basieren.

5. Computerimplementiertes Verfahren zum Bereitstellen (PROV-TF) einer trainierten Funktion (TF), umfassend:
- Erfassen (CAP-TPD) von Trainingsprojektionsabbildungen (TPD) eines Trainingsuntersuchungsobjekts, welche das Trainingsuntersuchungsobjekt aus verschiedenen Projektionsrichtungen abbilden,
wobei die Trainingsprojektionsabbildungen (TPD) simuliert oder mittels eines medizinischen Trainingsröntgengeräts aufgenommen sind,
- Bereitstellen (PROV-ITP) einer initialen Trainingsprojektionsmatrix (ITP) basierend auf einem statischen Trainingsmodell (STM) des Trainingsröntgengeräts,
- Bereitstellen einer weiteren Trainingsprojektionsmatrix (IFP) durch Anwenden der trainierten Funktion (TF) auf Eingabedaten,
wobei die Eingabedaten auf den Trainingsprojektionsabbildungen (TPD) und der initialen Trainingsprojektionsmatrix (ITP) basieren,
wobei die weitere Trainingsprojektionsmatrix (IFP) als Ausgabedaten der trainierten Funktion (TF) bereitgestellt wird,
- Rekonstruieren (RECO-TD) eines 3D-Trainingsdatensatzes (TD) aus den Trainingsprojektionsabbildungen (TPD) mittels der weiteren Trainingsprojektionsmatrix (IFP),
- Bestimmen (DET-BP) eines Bewertungsparameters (BP.TD) durch Anwenden einer Bildqualitätsmetrik und/oder einer Konsistenzmetrik auf den 3D-Trainingsdatensatz (TD),
- Anpassen (ADJ-TF) zumindest eines Parameters der trainierten Funktion (TF) basierend auf einem Vergleich des Bewertungsparameters (BP.TD) mit einem Referenzwert (RP),
- Bereitstellen (PROV-TF) der trainierten Funktion (TF).

6. Verfahren nach Anspruch 5,
wobei ein 3D-Vergleichsdatensatzes (VD) aus den Trainingsprojektionsabbildungen (TPD) mittels der initialen Trainingsprojektionsmatrix (ITP) rekonstruiert wird (RECO-VD),
wobei der Referenzwert (RP) durch Anwenden (DET-BP) der Bildqualitätsmetrik und/oder der Konsistenzmetrik auf den 3D-Vergleichsdatensatz (VD) bestimmt wird.

7. Verfahren nach Anspruch 6,
wobei jeweils eine initiale (ITP) und eine weitere Trainingsprojektionsmatrix (IFP) zu den verschiedenen Projektionsrichtungen bereitgestellt werden (PROV-ITP),
wobei die Eingabedaten der trainierten Funktion (TF) auf den initialen Trainingsprojektionsmatrizen (ITP) basieren,
wobei der 3D-Trainingsdatensatz (TD) durch Rekonstruktion (RECO-TD) aus den Trainingsprojektionsabbildungen (TPD) mittels der weiteren Trainingsprojektionsmatrizen (IFP) bereitgestellt wird.

8. Verfahren nach Anspruch 5 bis 7,
wobei eine Information zu dynamischen Bewegungsfreiheitsgraden des Trainingsröntgengeräts erfasst wird (CAP-DTM), welche einen latenten Trainingsraum (DTM) definiert,
wobei die Eingabedaten der trainierten Funktion (TF) zusätzlich auf dem latenten Trainingsraum (DTM) basieren.

9. Verfahren nach Anspruch 8,
wobei die Trainingsprojektionsabbildungen (TPD) für die verschiedenen Projektionsrichtungen basierend auf dem statischen Modell (STM) und dem latenten Trainingsraum (DTM) simuliert werden.

10. Verfahren nach einem der Ansprüche 5 bis 9,
wobei die Konsistenzmetrik eine epipolare Konsistenz und/oder eine Konsistenz von Vorwärtsprojektionen des 3D-Trainingsdatensatzes (TD) mit den Trainingsprojektionsabbildungen (TPD) bewertet und/oder wobei die Bildqualitätsmetrik eine Gesamtvariation des 3D-Trainingsdatensatzes (TD) bewertet.

11. Verfahren nach einem der Ansprüche 5 bis 10,
wobei die Eingabedaten der trainierten Funktion (TF) zusätzlich auf dem statischen Trainingsmodell (STM) des Trainingsröntgengeräts basieren.

12. Bereitstellungseinheit (PRVS), welche dazu ausgebildet ist, ein Verfahren nach einem der Ansprüche 1 bis 4 auszuführen.

13. Medizinisches Röntgengerät, umfassend eine Bereitstellungseinheit (PRVS) nach Anspruch 12,
wobei das Röntgengerät zur Aufnahme der Projektionsabbildungen (PD) des Untersuchungsobjekts (31) aus den verschiedenen Projektionsrichtungen ausgebildet ist.

14. Trainingseinheit (TRS), welche dazu ausgebildet ist, ein Verfahren nach einem der Ansprüche 5 bis 11 auszuführen.

15. Computerprogrammprodukt mit einem Computerprogramm, welches direkt in einen Speicher einer Bereitstellungseinheit (PRVS) ladbar ist, mit Programmabschnitten, um alle Schritte des Verfahrens nach einem der Ansprüche 1 bis 4 auszuführen, wenn die Programmabschnitte von der Bereitstellungseinheit (PRVS) ausgeführt werden und/oder welches direkt in einen Trainingsspeicher (TMU) einer Trainingseinheit (TRS) ladbar ist, mit Programmabschnitten, um alle Schritte des Verfahrens nach einem der Ansprüche 5 bis 11 auszuführen, wenn die Programmabschnitte von der Trainingseinheit (TRS) ausgeführt werden.
